(19) Europäisches Patentamt

European Patent Office

Office européen des brevets

(11) EP 1 054 681 B1

(12) **EUROPÄISCHE PATENTSCHRIFT**

(45) Veröffentlichungstag und Bekanntmachung des Hinweises auf die Patenterteilung:
**07.05.2003 Patentblatt 2003/19**

(21) Anmeldenummer: **98904169.4**

(22) Anmeldetag: **02.02.1998**

(51) Int Cl.7: **A61K 35/78**, A61K 31/43, A61K 31/545, A61K 45/06, A61P 31/04

(86) Internationale Anmeldenummer:
**PCT/EP98/00542**

(87) Internationale Veröffentlichungsnummer:
**WO 99/038521 (05.08.1999 Gazette 1999/31)**

(54) **VERWENDUNG VON WIRKSTOFFKOMBINATIONEN AUS ANTIBIOTIKA UND TERPENHALTIGEN PFLANZENEXTRAKTEN IN DER TIERMEDIZIN**

THE USE OF COMBINATIONS OF ACTIVE AGENTS CONSISTING OF ANTIBIOTICS AND PLANT EXTRACTS CONTAINING TERPENE IN VETERINARY MEDICINE

UTILISATION EN MEDECINE VETERINAIRE, DE COMBINAISONS DE PRINCIPES ACTIFS A BASE D ANTIBIOTIQUES ET D'EXTRAITS DE PLANTES CONTENANT DU TERPENE

(84) Benannte Vertragsstaaten:
**AT BE CH DE DK ES FI FR GB GR IE IT LI LU MC NL PT SE**

(43) Veröffentlichungstag der Anmeldung:
**29.11.2000 Patentblatt 2000/48**

(73) Patentinhaber: **BOEHRINGER INGELHEIM VETMEDICA GMBH**
**55218 Ingelheim am Rhein (DE)**

(72) Erfinder:
• **SCHLEICHER, Werner**
**D-55411 Bingen am Rhein (DE)**

• **SALAMON, Ernst**
**D-55218 Ingelheim (DE)**

(56) Entgegenhaltungen:
WO-A-84/01776     WO-A-84/04249
WO-A-91/10364     WO-A-97/30718
FR-A- 2 706 770     FR-A- 2 748 204

• CHEMICAL ABSTRACTS, vol. 126, no. 22, 2. Juni 1997 Columbus, Ohio, US; abstract no. 290666f, RHEE ET AL.: "Antimicrobial activities of a steam distillate of Leptospermum scoparium" Seite 391; Spalte r; XP002079097 & YAKHAK HOECHI, Bd. 41, Nr. 1, 1997, Seiten 132-138, SOUTH KOREA

## Beschreibung

Die vorliegende Erfindung betrifft neuartige Verwendungen von Wirkstoffkombinationen aus Antibiotika und terpenhaltigen Pflanzenextrakten in der Tiermedizin zur Behandlung der Mastitis und der Metritis bei landwirtschaftlichen Nutztieren sowie Kleintieren.

In den chemical abstracts Vol. 126, No. 22, 2. Juni 1997, abstract No. 290666f wird eine Hautcreme offenbart, welche Manukaöl in Kombination mit einem Gemisch aus dem Antibiotikum Gentamycinsulfat und dem Antimykotikum Clotrimazol sowie Hydrocortisonacetat enthält, und eine weitere Hautcreme, die neben Manukaöl als Wirkstoff das Antihistaminikum Diphenhydramin-Hydrochlorid sowie Hydrocortisonacetat enthält.

FR-A-2 706 770 beschreibt die antiseptische Wirkung von ätherischen Ölen wie Melaleuka Leucadendron, Thymus vulgaris, Eugenia caryophyllata und Eukalyptus globulus auf Tuberkelbazillen.

FR-A-2 748 204 offenbart die Anwendung von antiseptischen Terpinen-4-olhaltigen Ölen des Melaleuka-Typs für kosmetische und pharmazeutische Zwecke.

WO 91/10364 beschreibt einen desinfizierenden und auf Insekten abstoßend wirkenden Effekt von Teebaumöl. Daher könne besagtes Öl zur Prophylaxe von Mastitis eingesetzt werden, indem es als Spray oder Creme äußerlich auf ein Euter aufgetragen wird.

Der vorliegenden Erfindung liegt die Aufgabe zugrunde, bei der Behandlung bakteriell induzierter Erkrankungen den Einsatz des zur Behandlung erforderlichen Antibiotika mengenmäßig auf ein Minimum zu reduzieren, da mit derartigen Arzneimitteln unerwünschte Nebenwirkungen verbunden sind bzw. verbunden sein können. Als Beispiele seien bezüglich der Anwendung von Antibiotika im humanmedizinischen Bereich Überempfindlichkeitsreaktionen genannt. Insbesondere im veterinärmedizinischen Bereich führt die hohe Gabe von Antibiotika bei Tieren, die - oder deren Produkte - zum Verzehr bestimmt sind, beispielsweise zu hohen Wartezeiten, damit die Arzneimittel nicht durch den Menschen ungewollt aufgenommen werden und so zum Beispiel eine Resistenzbildung bei den Erregern gefördert wird.

Überraschenderweise wurde nun gefunden, daß die Kombination von Antibiotika - und besonders bevorzugt von Ampicillin, Cephalothin, Penicillin G und Spiramycin, die typische Vertreter der Aminopenicilline (Ampicillin), der Cephalosporine (Cephalothin), der Benzylpenicilline (Penicillin G) und der Makrolidantibiotika (Spiramycin) sind [M. Alexander, C.-J. Estler, F. Legler, Antibiotika und Chemotherapeutika, wissenschaftliche Verlagsgesellschaft mbH, Stuttgart 1995; Adam-Thoma, Antibiotika, Wissenschaftliche Verlagsgesellschaft mbH Stuttgart, 1994], mit terpenhaltigen Pflanzenextrakten, bevorzugt mit Extrakten aus Pflanzen der Gattungen Leptospermum und Melaleuca aus der Familie der Myrtaceae und besonders bevorzugt mit Teebaumöl (Extrakt von Melaleuca alternifolia) oder mit dem Öl des Kajeputbaums (Melaleuca leucadendra) zu einer überraschend hohen Potenzierung der antimikrobiellen Eigenschaften führt, die deutlich über einen additiven Effekt hinausgeht und somit eine Reduktion des Gehaltes des Antibiotikums ermöglicht. Dadurch werden auf der anderen Seite die eingangs geschilderten Nachteile, die mit der Verabreichung von Antibiotika verbunden sind, umgangen.

Die dabei vorzugsweise eingesetzten Extrakte aus den Blättern bzw. Pflanzenteilen der Pflanzengattungen Leptospermum und Melaleuca, deren natürliches Verbreitungsgebiet sich auf die subtropischen Küstenregionen von New South Wales beschränken, werden durch Wasserdampfdestillation bzw. Extraktion gewonnen. Dabei dienen besonders bevorzugt Blätter des australischen Teebaums (Melaleuca alternifolia) als Ausgangsprodukte.

Teebaumöl ist in Wasser praktisch unlöslich- jedoch mit den meisten organischen Lösungsmitteln gut mischbar - und besteht aus einem Vielstoffgemisch mit ca. 100 bekannten Bestandteilen. Es ist besonders reich an (+)-Terpinen-1-ol und enthält in kleineren Mengen folgende Monoterpene [R. Saller and J. Reichling, Deutsche Apotheker Zeitung 135 (1995) 40 und zit. Lit]:

α-Terpinen (ca. 10 %), γ-Terpinen (ca. 20 %) Terpinolen (ca. 4 %), α-Terpineol (3 %), α-Pinen, β-Pinen, Myrcen, α-Phellandren und 1,8-Cineol sowie die Sesquiterpene Aromadendren, Viridifloren und δ-Cadinen.

Als Antibiotika seien insbesondere als bevorzugte Wirkstoffe genannt:

Penicilline - insbesondere Penicillin G, Ampicillin, wie auch Amoxicillin und Bacampicillin - Cephalosporine, β-Lactam-Antibiotika, beispielsweise Oxacillin, Cloxacillin, Methicillin - oder Dihydropeptidasehemmer, Tetracycline - wie Oxytetracyclin-, Aminoglycoside - wie Gentamycin, Tobramycin, Neomycin, Kanamycin, Framycetin, Streptomycin u.a.-, Chloramphenicol, Florphenicol und Thiamphenicol, Lincomycine und Makrolid-Antibiotika, Polypeptid-Antibiotika, Chinolone (Gyrasehemmer), Nitroimidazole, sowie pflanzliche Antibiotika wie z. B. Perkolat aus Radix Umckaloabo.

Insbesondere für die äußerliche Anwendung werden bevorzugt Tetracyclin, Erythromycin, Fusidinsäure, Nebacetin, Gentamycin, Clindamycin, Framycetin, Neomycin, Chloramphenicol, Oxytetracyclin oder Sulfonamide eingesetzt.

Besonders bevorzugt werden Ampicillin, Cephalothin, Penicillin-G und Spiramycin.

Wie aus den experimentellen Befunden deutlich hervorgeht, zeigt Teebaumöl - alleine angewandt - keine inhibierende Wirkung auf das Wachstum von Staph. aureus in Nährlösung. (vgl. Experiment Nr. 1: Effekt von Teebaumöl auf das Wachstum von Staphylococcus aureus in Nährlösung!).

Noch deutlicher fällt das Ergebnis aus, wenn das Experiment in Milch durchgeführt wird (vgl. Experiment Nr. 2: Effekt von Teebaumöl auf das Wachstum von Staphylococcus aureus in normaler Milch!).

Aus beiden Experimenten geht im Gegenteil hervor, daß das Wachstum von Staphylococcus aureus durch die Anwesenheit von Teebaumöl vielmehr gefördert wird. Entsprechend gibt das dritte Experiment (Empfindlichkeit von Staphylococcus aureus gegenüber ausgewählten antibakteriellen Wirkstoffen) den inhibierenden Effekt typischer Vertreter der eingangs genannten Antibiotika-Klassen wieder. Alle Versuche belegen den erwarteten inhibierenden Einfluß dieses Wirkstoffs sowohl in Nährmedium als auch in Milch.

Die nachfolgenden Ergebnisse der Experimente 5 bis 7 belegen für jede der untersuchten antibakteriell wirkenden Substanzen eine deutliche gesteigerte Wirksamkeit gegenüber einer sowohl Kapsel-positiven als auch Kapsel-negativen Species von Staphylococcus aureus.

Dabei sticht die Verstärkung des inhibierenden Effekts ganz besonders bei der Kombination von Teebaumöl mit Spiramycin ins Auge.

Die nachstehend wiedergegebenen experimentellen Befunde liefern deutliche Belege für eine erfolgversprechende Behandlung von mikrobiell induzierten Erkrankungen durch die Verwendung von Wirkstoffkombinationen aus einem antimikrobiell wirkenden Mittel mit einem terpenhaltigen Pflanzenextrakt - insbesondere bei der Behandlung der Mastitis oder der Metritis bei Säugetieren, und hier vor allem bei bakteriell induzierten Erkrankungen bei landwirtschaftlichen Nutztieren - wie Schafen, Ziegen, Pferden, Rindern oder Schweinen - sowie bei Kleintieren - wie Hunde Katzen und Kaninchen.

Alle genannten Wirkstoffe können entweder allein oder in Kombination mit anderen Wirkstoffen sowie zusätzlich mit weiteren Hilfsstoffen in die erfindungsgemäße Wirkstoffkombination Eingang finden. Ebenso können die terpenhaltigen Extrakte als Einzelextrakt wie auch als Extraktgemisch in der erfindungsgemäßen Wirkstoffkombination Verwendung finden. Die erfindungsgemäßen Wirkstoffkombinationen können in Form von Cremes, Salben, Lotionen, W/O- oder O/W-Emulsion oder Aerosolschäumen verabreicht werden. Sie können aber auch oral in Form von Tabletten, Kapseln, beispielsweise Hart- oder Weichgelatine-Kapseln oder Dragees appliziert werden. Die Herstellung derartiger Arzneiformen ist an sich aus dem Stand der Technik wohlbekannt.

In der Veterinärmedizin kann die erfindungsgemäße Wirkstoffkombination neben der Behandlung der Metritis insbesondere bei der Behandlung von Mastitiden bei Milchkühen und Sauen vorteilhaft angewandt werden, wobei als Zubereitungen zweckmäßigerweise - neben Cremes, Salben, Lotionen oder Emulsionen - Aerosolschäume oder die Form des Bolus' für die Darreichung in Frage kommen.

Im folgenden sind einige - beispielhaft ausgewählte und typische Zubereitungen - Schaumdruckgas-Zusammensetzungen zur Erzeugung von Aerosolschäumen genannt. Diese Zusammensetzungen bestehen im wesentlichen - neben dem terpenhaltigen Pflanzenextrakt und der antimikrobiell wirkenden Substanz aus einem sog. Vehikel, Antioxidantien zur Stabilisierung der Komponenten gegen Sauerstoff-Einfluß, Schaumbildnern, Emulgatoren, Konservierungsmitteln und einem Treibgas.

Die Verabreichung solcher Aerosolschäume kann entweder direkt als fixe Kombination von Teebaumöl mit Antibiotika erfolgen oder durch eine aufeinanderfolgende Anwendung einer beliebigen Antibiotika-Zubereitung (in Form von Salben, Schäumen etc.) und einer Schaumdruckgas-Zubereitung, die Teebaumöl allein enthält. Durch die zuletztgenannte Form der Anwendung kann eine bessere Verteilung im Zielorgan zusammen mit einer Wirksamkeitssteigerung erreicht werden ("Booster"-Effekt).

Als Anwendungsbeispiele hierfür sind nachstehend zwei dieser "Booster"-Rezepturen (3. und 4.) aufgeführt.

Das Vehikel kann aus Wasser und/oder öligen Komponenten gebildet werden.

Als ölige Komponenten eignen sich hierzu alle aus dem Stand der Technik für die Herstellung von Pharmazeutika bekannten Wirkstoffe wie z.B. vorzugsweise Pflanzenöle- wie z.B. Baumwollsaat-, Erdnuß-, Mais-, Raps-, Sesam- und Sojaöl - oder Triglyceride mittlerer Kettenlänge - beispiels fraktioniertes Koskosnußöl - oder Isopropylmyristat, -palmitat oder Mineralöle oder Ethyloleat.

Als Antioxidantien kommen alle aus dem Stand der Technik bekannten Antioxidantien in Frage, wie bevorzugterweise a-Tocopherol, Butylhydroxytoluol (BHT) oder Butylhydroxyanisol (BHA).

Als Schaumbildner können alle arzneimittelrechtlich zulässigen und aus dem Stand der Technik bekannten Schaumbildner - bevorzugt Polyoxyethylensorbitanester verschiedener Fettsäuren (Polysorbate) - eingesetzt werden.

Als Emulgatoren werden neben den aus dem Stand der Technik bekannten Emulgatoren vorzugsweise Polyoxyethylenderivate des Rizinusöls oder Polyoxyethylenalkylether eingesetzt.

Die vorgenannten Voraussetzungen gelten auch für die Konservierungsmittel, von diesen vorzugsweise Konservierungsmittel aus der Gruppe der PHB-Ester - z.B. Mischung von PHB-Methyl- mit PHB-Propylester -, quarternäre Ammoniumverbindungen - wie z.B. Benzalkoniumchlorid -, Phenol, Chlorbutanol, Chlorkresol, Ethylalkohol, Thiomersal, Phenylquecksilbersalze-wie z.B. Nitrate, Borate etc. -oder Benzoe- und Sorbinsäure sowie deren Salze.

Als Treibgase eignen sich alle für eine Anwendung auf medizinischem Gebiet zulässigen und aus dem Stand der Technik bekannten Treibgase - wie z.B. $CO_2$, $N_2O$, $N_2$, Propan / Butan - Mischungen, Isobutan, Chlorpentafluorethan ($CClF_2$- $CF_3$), Octafluorcyclobutan ($C_4F_8$).

Einige beispielhafte Darreichungsformen sind dem experimentellen Teil angefügt.

Die beschriebene Erfindung wird nunmehr durch die folgenden Beispiele erläutert. Verschiedenartige, andere Ausgestaltungen werden für den Fachmann aus der vorliegenden Beschreibung ersichtlich. Es wird jedoch ausdrücklich darauf hingewiesen, daß die Beispiele und die Beschreibung lediglich zur Erläuterung vorgesehen und nicht als Einschränkung der Erfindung anzusehen sind.

I. Experimentelle Ergebnisse:

Vorbemerkungen:

Die Empfindlichkeitstests und die Experimente, die mit Wirkstoffkombinationen - unter Verwendung des sogenannten Checkerboard Designs - in Brühe (Nährmedium) und Milch gemacht wurden, wurden mit Mikrotitrationsplatten durchgeführt. - Dabei wurden jeweils drei Kapsel-positive und drei Kapselnegative Stämme von Staphylococcus aureus eingesetzt. Der sogenannte MIC-Wert wurde mit fluorometrischen Verfahren bestimmt (in Milch).

Dabei bedeutet der MIC-Wert die minimale Konzentration, bei der ein inhibierender Effekt nachgewiesen werden kann.

Die Konzentration der Maßlösung des eingesetzten Teebaumöls betrug 4 % vol/vol.

Die höchste Konzentration, die in den Tests eingesetzt wurde, war 1/10 der ursprünglichen Maßlösung, d.h.: 0,4 Vol.-%.

Der MIC wird in den folgenden Beispielen als diejenige niedrigste Wirkstoffkonzentration definiert, die ein bakteriell bedingtes Ansteigen der Trübung - in Nährlösung - oder ein Ansteigen der Fluorescenz - in Milch - inhibiert. Es bedeuten:

FIC (Fraktionell inhibierende Konzentration) =

A/MICa, worin A den MIC-Wert der antibakteriell wirkenden Substanz in

Gegenwart der höchsten Konzentration des Teebaumöls bedeutet und

MICa den MIC-Wert der antibakteriell wirkenden Substanz alleine

bedeutet.

Somit bedeutet ein FIC-Wert < 0,5 eine Verstärkung der Wirksamkeit durch das jeweilige Teebaumöl (Tböl), während FIC-Werte von > 1 einen Antagonismus implizieren.

Miglyol [Fiedler, H. P., Lexikon der Hilfsstoffe für Pharmazie, Kosmetik und angrenzende Gebiete, 4. Auflage, Editio Cantor Verlag, Aulendorf 1996, Vol. II], als emulgierte Maßlösung mit 10 % v/v zeigt keinen inhibierenden Effekt auf das Wachstum von S. aureus in Milch (bei einer maximalen Konzentration von 1 %).

1 Effekt von Teebaumöl (Tböl) auf das Wachstum von Staphylococcus aureus in Nährlösung (ISB). Die Werte sind dargestellt als gemittelter Wert der minimalen Inhibitionskonzentration von vier Replikaten

## MIC in ISB (mg/ml)

| | Kapsel-negative Staph. aureus | | | Kapsel-positive Staph. aureus | | |
|---|---|---|---|---|---|---|
| | SaA | SaB | SaC | SaD | SaE | SaF |
| Tböl | 4 | 4 | >4 | >4 | >4 | 2 |

2 Effekt von Teebaumöl auf das Wachstum von Staphylococcus aureus in normaler Milch. Jeder Stamm wurde in vier Replikaten getestet.

## MIC in Milch (mg/ml)

### Kapsel-negative Staph. aureus    Kapsel-positive Staph. aureus

|  | SaA | SaB | SaC | SaD | SaE | SaF |
|---|---|---|---|---|---|---|
| Tböl | >4 | >4 | >4 | >4 | >4 | >4 |

3 Suszeptibilität von Staphylococcus aureus gegenüber ausgewählten antibakteriellen Mitteln in Nährmedium (ISB) und Milch. Die Werte sind dargestellt als durchschnittliche minimale Hemmkonzentration (MIC) (+ Standardabweichung) von drei Kapsel-positiven bzw. drei Kapselnegativen Stämmen. Jeder Stamm wurde in 4 Replikaten getestet.

## MIC in ISB (µg/ml)

### Kapsel-negative Staph. aureus    Kapsel-positive Staph. aureus

|  | ISB | Milch | ISB | Milch |
|---|---|---|---|---|
| Ampicillin | $0,198 \pm 0.018$ (0,125 - 0,25) | $0,104 \pm 0.009$ (0,063 - 0,125) | $0,188 \pm 0.019$ (0.125 - 0,25) | $0,12 \pm 0,005$ (0,063 - 0,125) |
| Cephalothin | $0,188 \pm 0.019$ (0.125 - 0.25) | $0,167 \pm 0,018$ (0.125 - 0,25) | $0,188 \pm 0,019$ (0,125 - 0,25) | 0,25 |
| Penicillin G | $0,033 \pm 0,004$ (0,025 - 0,05) | $0,031 \pm 0,005$ (0,013 - 0,05) | $0,035 \pm 0,004$ (0,025 - 0,05) | $0,022 \pm 0,002$ (0,013 - 0,025) |
| Spiramycin | $15 \pm 1,5$ (10 - 20) | $25,52 \pm 4,82$ (6,25 - 50) | $15,83 \pm 1,49$ (10 - 20) | $25 \pm 3,77$ (12,5 - 50) |

4 Effekt von Teebaumöl (Tböl) auf die Wirksamkeit von Ampicillin in Nährmedium (ISB) und Milch. FIC = 0,5 additive Wirkung, < 0,5 Potenzierung und >2 Antagonismus. Staph. aureus A - C sind Kapsel-negativ und Staph. aureus D - F Kapsel-positiv.

## Ampicillin FIC

| | | SaA | SaB | SaC | SaD | SaE | SaF | Häufigkeit der Potenzierung* | Mittelwert** |
|---|---|---|---|---|---|---|---|---|---|
| Tböl | ISB | 0,063 | 0,125 | 0,125 | 0,125 | 0,25 | 0,25 | 6/6 | 0,14/0,13 |
| | Milch | 0 | 2 | 0 | 2 | 0 | 0 | -(2/6) | 2 |

* Verhältnis der Potenzierung oder Verhältnis der antagonistischen Wirkung mit "-" Zeichen. Dasselbe gilt für die folgenden Tabellen 5 - 7.

** Geometrische Mittel/Mittel - wo zutreffend; dasselbe gilt für die folgenden Tabellen 5 - 7.

5 Effekt von Teebaumöl auf die Wirksamkeitspotenz von Cephalothin in Brühe (ISB) und Milch.
FIC = 0,5 bedeutet additive Wirkung, < 0,5 Potenzierung und > 2 Antagonismus.
Staph. aureus A - C sind Kapsel-negativ und Staph. aureus D - F Kapsel-positiv.

## Cephalothin FIC

| | | SaA | SaB | SaC | SaD | SaE | SaF | Häufigkeit der Potenzierung* | Mittelwert** |
|---|---|---|---|---|---|---|---|---|---|
| Tböl | ISB | 0,063 | 0,125 | 0,125 | 0,063 | 0,5 | 0,25 | 6/6 | 0,14/0,13 |
| | Milch | 0 | 0 | 0 | 0 | 0 | 0,5 | 1/6 | 0,5 |

* Verhältnis der Potenzierung oder Verhältnis der antagonistischen Wirkung mit - Zeichen. Dasselbe gilt für die folgenden Tabellen 5 - 7.

** Geometrisches Mittel/Mittel - wo zutreffend; dasselbe gilt für die folgenden Tabellen 5 - 7.

6 Einfluß von Teebaumöl auf die Wirksamkeit von Penicillin G in Nährmedium (ISB) und Milch.
FIC = 0,5 bedeutet Zusatz, < 0,5 Potenzierung und > 2 Antagonismus.
Staph. aureus A - C sind Kapsel-negativ und Staph. aureus D - F Kapsel-positiv.

## Penicillin G  FIC

| | | SaA | SaB | SaC | SaD | SaE | SaF | Häufigkeit der Potenzierung* | Mittelwert** |
|---|---|---|---|---|---|---|---|---|---|
| Tböl | ISB | 0,063 | 0,125 | 0,125 | 0,25 | 0,125 | 0,25 | 6/6 | 0,14/0,13 |
| | Milch | 0 | 0 | 0 | 2 | 0 | 0 | -(1/6) | 2 |

7 Einfluß von Teebaumöl auf die Wirksamkeit von Spiramycin in Nährmedium (ISB) und Milch. Staph. aureus A - C sind Kapsel-negativ und Staph. aureus D - F Kapsel-positiv.

## Spiramycin FIC

| | | SaA | SaB | SaC | SaD | SaE | SaF | Häufigkeit der Potenzierung* | Mittelwert** |
|---|---|---|---|---|---|---|---|---|---|
| Tböl | ISB | 0,063 | 0,063 | 0,125 | 0,063 | 0,25 | 0,125 | 6/6 | 0,1/0.09 |
| | Milch | 0,5 | 0 | 0,5 | 0 | 0 | 0,5 | 3/6 | 0,5 |

II. Typische Beispielrezepturen bzw Zusammensetzungen von Schaum-Druckgaspackungen

1. Wäßrige Suspension von Teebaumöl in Kombination mit einem Antibiotikum

| Komponente | Anteil [%] | Bereich [%] |
|---|---|---|
| Teebaumöl | 0,5 | 0,01 - 20,0 |
| Antibiotikum | 5,0 | 0,1 - 15,0 |
| **Schaumbildner** | 2,0 | 0,1 - 10,0 |
| Emulgator | 3,0 | 0,1 - 20,0 |
| Antioxidans | 0,5 | 0,01 - 5,0 |
| Konservans | 0,5 | 0,01 - 2,0 |
| Wasser | 63,5 | 10,0 - 90,0 |
| Treibgas | 25,0 | 1,0 - 40,0 |
| **Summe** | **100,0** | |

2. Ölige Suspension von Teebaumöl in Kombination mit einem Antibiotikum

| Komponente | Anteil [%] | Bereich [%] |
|---|---|---|
| Teebaumöl | 0,5 | 0,01 - 20,0 |
| Antibiotikum | 5,0 | 0,1 - 15,0 |

(fortgesetzt)

| Komponente | Anteil [%] | Bereich [%] |
|---|---|---|
| **Schaumbildner** | 5,0 | 0,1 - 10,0 |
| Emulgator | 5,0 | 0,1 - 20,0 |
| Antioxidans | 0,5 | 0,01 - 5,0 |
| Konservans | 0,5 | 0,01 - 2,0 |
| öliges Vehikel | 58,5 | 10,0 - 90,0 |
| Treibgas | 25,0 | 1,0 - 40,0 |
| **Summe** | **100,0** | |

3. "Booster" (Schaumzusammensetzung), wäßrig, mit Teebaumöl allein

| Komponente | Anteil [%] | Bereich [%] |
|---|---|---|
| Teebaumöl | 0,5 | 0,01 - 20,0 |
| **Schaumbildner** | 2,0 | 0,1 - 10,0 |
| Emulgator | 3,0 | 0,1 - 20,0 |
| Antioxidans | 0,5 | 0,01 - 5,0 |
| Konservans | 0,5 | 0,01 - 2,0 |
| Wasser | 68,5 | 10,0 - 90,0 |
| Treibgas | 25,0 | 1,0 - 40,0 |
| **Summe** | **100,0** | |

4. "Booster", ölig, mit Teebaumöl allein

| Komponente | Anteil [%] | Bereich [%] |
|---|---|---|
| Teebaumöl | 0,5 | 0,01 - 20,0 |
| **Schaumbildner** | 5,0 | 0,1 - 10,0 |
| Emulgator | 5,0 | 0,1 - 20,0 |
| Antioxidans | 0,5 | 0,01 - 5,0 |
| Konservans | 0,5 | 0,01 - 2,0 |
| öliges Vehikel | 63,5 | 10,0 - 90,0 |
| Treibgas | 25,0 | 1,0 - 40,0 |
| **Summe** | **100,0** | |

**Patentansprüche**

1. Verwendung einer Wirkstoffkombination aus einem terpenhaltigen Pflanzenextrakt und einem Antibiotikum zur Herstellung eines veterinärmedizischen Arzneimittels zur Erzeugung eines Aerosolschaums zur Behandlung der Mastitis und/oder Metritis im Zielorgan.

2. Verwendung einer Wirkstoffkombination nach Anspruch 1 zur Herstellung eines veterinärmedizinischen Arzneimittels zur Behandlung der Mastitis.

3. Verwendung einer Wirkstoffkombination nach Anspruch 1 zur Herstellung eines veterinärmedizinischen Arzneimittels zur Behandlung der Metritis.

4. Verwendung einer Wirkstoffkombination nach einem der Ansprüche 1 bis 3, **dadurch gekennzeichnet, daß** das Antibiotikum ein Wirkstoff aus der Gruppe der Aminopenicilline, Cephalosporine, Benzylpenicilline und/oder Makrolidantibiotika ist.

5. Verwendung einer Wirkstoffkombination nach einem der Ansprüche 1 bis 3, **dadurch gekennzeichnet**, das das Antibiotikum ein Wirkstoff aus der Gruppe Penicillin G, Ampicillin, Amoxicillin, Bacampicillin, Gentamycin, Tobramycin, Neomycin, Kanamycin, Framycetin, Streptomycin, Chloramphenicol, Florphenicol, Thiamphenicol, Oxacillin, Cloxacillin, Methicillin sowie Perkolat aus Radix Umckaloabo ist.

6. Verwendung einer Wirkstoffkombination für die Herstellung eines Arzneimittels nach einem der Ansprüche 1 bis 3, **dadurch gekennzeichnet, daß** das Antibiotikum ein Wirkstoff aus der Gruppe Tetracyclin, Erythromycin, Fusidinsäure, Nebacetin, Gentamycin, Clindamycin, Framycetin, Neomycin, Chloramphenicol, Oxytetracyclin und/oder ein Sulfonamid ist.

7. Verwendung einer Wirkstoffkombination nach einem der Ansprüche 1 bis 3, **dadurch gekennzeichnet, daß** das Antibiotikum ein Wirkstoff aus der Gruppe Ampicillin, Cephalothin, Penicillin-G und/oder Spiramycin ist.

8. Verwendung einer Wirkstoffkombination nach einem Ansprüche 1 bis 7, **dadurch gekennzeichnet, daß** der terpenhaltige Pflanzenextrakt ein Extrakt aus Pflanzenteilen von einem oder mehreren Gewächsen aus der Familie der Myrtaceae ist.

9. Verwendung einer Wirkstoffkombination nach Anspruch 8, **dadurch gekennzeichnet, daß** der Pflanzenextrakt ein Extrakt aus Pflanzenteilen von Gewächsen aus der Gruppe Leptospermum und/oder Melaleuca ist.

10. Verwendung einer Wirkstoffkombination nach Anspruch 8 oder 9, **dadurch gekennzeichnet, daß** der Pflanzenextrakt ein Extrakt aus Pflanzenteilen von Gewächsen aus der Gruppe Melaleuca altemifolia (Teebaum) und/oder Melaleuca leucadendra ist.

11. Verwendung nach einem der Ansprüche 1 bis 10, **dadurch gekennzeichnet, daß** der terpenhaltige Pflanzenextrakt Teebaumöl ist und in dem Arzneimittel in einer Menge von 0,01-20 Gew.% vorliegt.

12. Verwendung nach einem der Ansprüche 1 bis 11, **dadurch gekennzeichnet, daß** die Menge des Antibiotikums in dem Arzneimittel 0,1-15 Gew.% beträgt.

13. Verwendung nach einem der Ansprüche 1 bis 12, **dadurch gekennzeichnet, daß** der Aerosolschaum eine wäßrige Suspension der Wirkstoffkombination ist mit einem Wasseranteil von 10-90 Gew.%.

14. Verwendung nach einem der Ansprüche 1 bis 12, **dadurch gekennzeichnet, daß** der Aerosolschaum eine ölige Suspension der Wirkstoffkombination ist, mit einem Anteil des öligen Vehikels von 10-90 Gew.%.

15. Verwendung einer Wirkstoffkombination nach einem der Ansprüche 1 bis 14, **dadurch gekennzeichnet, daß** das Arzneimittel in Form einer Schaum-Druckgas-Einheit (Booster Zubereitung) vorliegt.

16. Verwendung einer Wirkstoffkombination nach einem Ansprüche 1 bis 15 in Form einer Booster-Zubereitung, **dadurch gekennzeichnet, daß** die Wirkstoffkombination aus einer Antibiotika-Zubereitung besteht und einer Schaum-Druckgas-Einheit, die als Wirkstoff Teebaumöl aber kein Antibiotikum beinhaltet, wobei die Schaum-Druckgas-Einheit unmittelbar nach Verabreichung derAntibiotikum-Zubereitung zur Anwendung kommt, um die Wirkstoffverteilung zu verbessern und damit die Wirksamkeit zu erhöhen.

17. Verwendung einer Wirkstoffkombination nach einem der Ansprüche 1 bis 16, zur Herstellung eines Arzneimitels zur Behandlung von landwirtschaftlichen Nutztieren wie Schafen, Ziegen, Pferde, Schweinen und Rindern sowie Kleintieren wie Hunde, Katzen und Kaninchen.

**Claims**

1. Use of a combination of active agents consisting of a plant extract containing terpene and an antibiotic, for the production of a veterinary medical preparation for producing an aerosol foam for treating mastitis and/or metritis in the target organ.

2. Use of a combination of active agents according to claim 1 for the production of a veterinary medical preparation for treating mastitis.

3. Use of a combination of active agents according to claim 1 for the production of a veterinary medical preparation for treating metritis.

4. Use of a combination of active agents according to one of claims 1 to 3, **characterised in that** the antibiotic is an active substance selected from among the aminopenicillins, cephalosporins, benzylpenicillins and/or macrolide antibiotics.

5. Use of a combination of active agents according to one of claims 1 to 3, **characterised in that** the antibiotic is an active substance selected from among penicillin G, ampicillin, amoxycillin, bacampicillin, gentamycin, tobramycin, neomycin, kanamycin, framycetin, streptomycin, chloramphenicol, florphenicol, thiamphenicol, oxacillin, cloxacillin, methicillin and percolate from Radix Umckaloabo.

6. Use of a combination of active agents for preparing a pharmaceutical composition according to one of claims 1 to 3, **characterised in that** the antibiotic is an active substance selected from among tetracycline, erythromycin, fusidic acid, nebacetin, gentamycin, clindamycin, framycetin, neomycin, chloramphenicol, oxytetracycline and/or a sulphonamide.

7. Use of a combination of active agents according to one of claims 1 to 3, **characterised in that** the antibiotic is an active substance selected from among ampicillin, cephalothin, penicillin G and/or spiramycin.

8. Use of a combination of active agents according to one of claims 1 to 7, **characterised in that** the terpene-containing plant extract is an extract from plant parts from one or more plants of the Myrtaceae family.

9. Use of a combination of active agents according to claim 8, **characterised in that** the plant extract is an extract from parts of plants of the Leptospermum and/or Melaleuca group.

10. Use of a combination of active agents according to claim 8 or 9, **characterised in that** the plant extract is an extract from parts of plants selected from among Melaleuca alternifolia (tea tree) and/or Melaleuca leucadendra.

11. Use according to one of claims 1 to 10, **characterised in that** the plant extract containing terpene is tea tree oil and is present in the pharmaceutical composition in an amount of from 0.1-20% by weight.

12. Use according to one of claims 1 to 11, **characterised in that** the amount of antibiotic in the pharmaceutical composition is from 0.1 to 15% by weight.

13. Use according to one of claims 1 to 12, **characterised in that** the aerosol foam is an aqueous suspension of the combination of active agents with a water content of from 10-90% by weight.

14. Use according to one of claims 1 to 12, **characterised in that** the aerosol foam is an oily suspension of the combination of active agents, with a content of oily carrier of from 10-90% by weight.

15. Use of a combination of active agents according to one of claims 1 to 14, **characterised in that** the pharmaceutical composition is present in the form of a pressurised foam unit (booster preparation).

16. Use of a combination of active agents according to one of claims 1 to 15 in the form of a booster preparation, **characterised in that** the combination of active agents consists of an antibiotic preparation and a pressurised foam unit containing as active substance tea tree oil but no antibiotic, the pressurised foam unit being used immediately after the administration of the antibiotic preparation, in order to improve the distribution of the active substance and hence increase its activity.

**17.** Use of a combination of active agents according to one of claims 1 to 16 for preparing a pharmaceutical composition for the treatment of agricultural animals such as sheep, goats, horses, pigs and cattle as well as small animals such as dogs, cats and rabbits.

## Revendications

**1.** Utilisation d'une combinaison de principes actifs consistant en un extrait végétal contenant des terpènes et en un antibiotique pour la préparation d'un médicament de médecine vétérinaire pour la production d'une mousse d'aérosol pour le traitement de la mastite et/ou de la métrite dans l'organe cible.

**2.** Utilisation d'une combinaison de principes actifs selon la revendication 1 pour la préparation d'un médicament de médecine vétérinaire pour le traitement de la mastite.

**3.** Utilisation d'une combinaison de principes actifs selon la revendication 1 pour la préparation d'un médicament de médecine vétérinaire pour le traitement de la métrite.

**4.** Utilisation d'une combinaison de principes actifs selon l'une des revendications 1 à 3 **caractérisée en ce que** l'antibiotique est un principe actif du groupe des aminopénicillines, des céphalosporines, des benzylpénicillines et/ou des antibiotiques macrolides.

**5.** Utilisation d'une combinaison de principes actifs selon l'une des revendications 1 à 3 **caractérisée en ce que** l'antibiotique est un principe actif du groupe de la pénicilline G, de l'ampicilline, de l'amoxicilline, de la bacampicilline, de la gentamycine, de la tobramycine, de la néomycine, de la kanamycine, de la framycétine, de la streptomycine, du chloramphénicol, du florphénicol, du thiamphénicol, de l'oxacilline, de la cloxacilline, de la méthicilline ainsi que d'un percolat de Radix Umckaloabo.

**6.** Utilisation d'une combinaison de principes actifs pour la préparation d'un médicament selon l'une des revendications 1 à 3 **caractérisée en ce que** l'antibiotique est un principe actif du groupe de la tétracycline, de l'érythromycine, de l'acide fusidique, de la nébacétine, de la gentamycine, de la clindamycine, de la framycétine, de la néomycine, du chloramphénicol, de l'oxytétracycline et/ou un sulfamide.

**7.** Utilisation d'une combinaison de principes actifs selon l'une des revendications 1 à 3 **caractérisée en ce que** l'antibiotique est un principe actif du groupe de l'ampicilline, de la céphalothine, de la pénicilline G et/ou de la spiramycine.

**8.** Utilisation d'une combinaison de principes actifs selon l'une des revendications 1 à 7 **caractérisée en ce que** l'extrait végétal contenant des terpènes est un extrait de parties végétales d'une ou plusieurs plantes de la famille des myrtacées.

**9.** Utilisation d'une combinaison de principes actifs selon la revendication 8 **caractérisée en ce que** l'extrait végétal est un extrait de parties végétales de plantes du groupe de Leptospermum et/ou Melaleuca.

**10.** Utilisation d'une combinaison de principes actifs selon la revendication 8 ou 9 **caractérisée en ce que** l'extrait végétal est un extrait de parties végétales de plantes du groupe de Melaleuca alternifolia (théier) et/ou de Melaleuca leucadendra.

**11.** Utilisation selon l'une des revendications 1 à 10 **caractérisée en ce que** l'extrait végétal contenant des terpènes est l'huile de théier et est présent dans le médicament en une quantité de 0,01-20 % en masse.

**12.** Utilisation selon l'une des revendications 1 à 11 **caractérisée en ce que** la quantité d'antibiotique dans le médicament est 0,1-15 % en masse.

**13.** Utilisation selon l'une des revendications 1 à 12 **caractérisée en ce que** la mousse d'aérosol est une suspension aqueuse de la combinaison de principes actifs avec une proportion d'eau de 10-90 % en masse.

**14.** Utilisation selon l'une des revendications 1 à 12 **caractérisée en ce que** la mousse d'aérosol est une suspension huileuse de la combinaison de principes actifs avec une proportion de véhicule huileux de 10-90 % en masse.

**15.** Utilisation d'une combinaison de principes actifs selon l'une des revendications 1 à 14 **caractérisée en ce que** le médicament est sous forme d'une unité mousse-gaz comprimé (préparation booster).

**16.** Utilisation d'une combinaison de principes actifs selon l'une des revendications 1 à 15 sous forme d'une préparation booster **caractérisée en ce que** la combinaison de principes actifs consiste en une préparation d'antibiotique et une unité mousse-gaz comprimé qui contient comme principe actif de l'huile de théier mais pas d'antibiotique, où l'unité mousse-gaz comprimé est utilisée immédiatement après l'administration de la préparation d'antibiotique pour améliorer la distribution des principes actifs et augmenter ainsi l'efficacité.

**17.** Utilisation d'une combinaison de principes actifs selon l'une des revendications 1 à 16 pour la préparation d'un médicament pour le traitement des animaux utiles en agriculture comme les moutons, les chèvres, les chevaux, les porcs et les bovins ainsi que des petits animaux comme les chiens, les chats et les lapins.